(19) Europäisches Patentamt
European Patent Office
Office européen des brevets

(11) Publication number: **0 358 508 A2**

(12) # EUROPEAN PATENT APPLICATION

(21) Application number: 89309089.4

(22) Date of filing: 07.09.89

(51) Int. Cl.[5]: C 07 H 19/01
A 61 K 31/70, C 12 P 19/62

(30) Priority: 08.09.88 US 242019 08.09.88 US 242020

(43) Date of publication of application:
14.03.90 Bulletin 90/11

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Borris, Robert P.
134 Fox Hollow Road
Glen Gardner New Jersey 08826 (US)

Wicker, Linda S.
2096 Arrowwood Drive
Westfield New Jersey 07090 (US)

Zink, Deborah L.
37 Blenheim Road
Manalapan New Jersey 07726 (US)

Lien, Thoai Hung
74 Longfellow Drive
Colonia New Jersey 07067 (US)

(74) Representative: Hesketh, Alan, Dr. et al
European Patent Department Merck & Co., Inc. Terlings
Park Eastwick Road
Harlow Essex, CM20 2QR (GB)

(54) **Novel immunosuppressant compound.**

(57) Described is a new immunosuppressant, "Tsukubamycin A", being the C-9 dihydro analog of FK-506, and a new process for preparing the immunosuppressant, "Tsukubamycin B", being the C-21 propyl analog of FK-506, produced as by-products under fermentation conditions for producing FK-506 utilizing the microorganism, Streptomyces tsukubaensis No. 9993 (FERM BP-927). The macrolide immunosuppressants are useful in preventing human host rejection of foreign organ transplants, e.g. bone marrow and heart transplants.

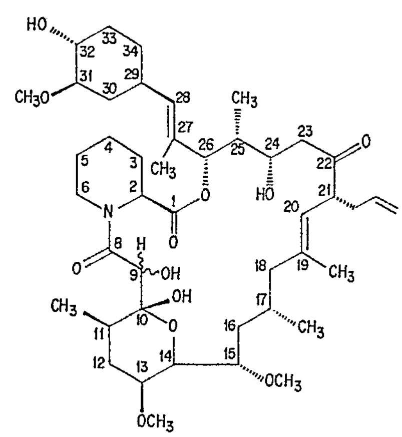

FIG. I

EP 0 358 508 A2

## Description

# NOVEL IMMUNOSUPPRESSANT COMPOUND

## BACKGROUND OF THE INVENTION

### 1. Field of the Invention

This invention relates to a new immunosuppressant agent, "Tsukubamycin A", the C-9 dihydro analog of FK-506, and a new process for producing the immunosuppressant agent "Tsukubamycin B", being the C-21 propyl analog of FK-506, obtained as by-products in the fermentation process for producing FK-506 utilizing the microorganism Streptomyces tsukubaensis No. 9993 (FERM BP-927). The process involves culturing the microorganism, under conditions which also produce FK-506. Also disclosed is a method of use in a human host for treatment of autoimmune diseases, infectious diseases and/or prevention of organ transplant rejections.

### 2. Brief Description of Disclosures in the Art

In 1983, the US FDA licensed cyclosporin, an extremely effective anti-rejection drug that revolutionized the field of organ transplant surgery. The drug acts by inhibiting the body's immune system from mobilizing its vast arsenal of natural protecting agents to reject the transplant's foreign protein.

As effective as the drug is in fighting transplantation rejection, it suffers drawbacks in causing kidney failure, liver damage and ulcers which in many cases can be very severe.

EPO Publication No. 0184162 to Fujisawa, hereby incorporated by reference, describes a new macrolide immunosuppressant FK-506 which is reputed to be 100 times more effective than cyclosporin. The macrolide is produced by fermentation of a particular strain of Streptomyces tsukubaensis No. 9993 (FERM BP-927). Also described are the closely related macrolide immunosuppressants FK-525, produced by the same microorganism, and FK-520 and FK-523, produced by S. hygroscopicus subsp. yakushimaensis.

USP 3,244,592 to T. Arai describes the culturing of Streptomyces hygroscopicus var. ascomyceticus to produce the antifungal "ascomycin".

There is, however, no description in the literature of the production of any immunosuppressive agents, which substantially lack the side effects of cyclosporin.

Newer, safer drugs exhibiting less side effects are constantly being searched for in the field.

## BRIEF DESCRIPTION OF THE FIGURES

Figure 1 illustrates the assigned molecular structure for "Tsukubamycin A", being the C-9 dihydro analog of FK-506.

Figure 2 is the proton NMR spectrum of "Tsukubamycin A" taken in $CDCl_3$ at 300 MHz.

Figure 3 illustrates the assigned structure of "Tsukubamycin B."

Figure 4 is the proton nuclear magnetic resonance spectrum of "Tsukubamycin B" taken in $CDCl_3$ at 300 MHz.

Figure 5 is the $^{13}C$ nuclear magnetic resonance spectrum of "Tsukubamycin B" taken in $CDCl_3$ at 75 MHz.

## SUMMARY OF THE INVENTION

It has been found that a new immunosuppressant, "Tsukubamycin A", being the C-9 dihydro analog of FK-506, and "Tsukubamycin B" being the C-21 propyl analog of FK-506, can be obtained as by-products in the fermentation of the microorganism Streptomyces tsukubaensis No. 9993, (FERM BP-927) under submerged aerobic conditions for producing FK-506 in an aqueous carbohydrate medium, containing a nitrogen nutrient, said conditions being conducted at a pH of about 7.

The resultant "Tsukubamycins A and B" exhibit immunosuppressive activity, i.e., positive inhibition of T-cell activation, as demonstrated by the calcium ionophore (ionomycin) plus phorbol myristate acetate (PMA) induced T-cell stimulation assay, also referred to herein as the "T-cell proliferation assay".

The principle of this assay is to measure the proliferation of mouse T lymphocytes stimulated with the combination of ionomycin plus PMA. A positive sample in this assay will inhibit T-cell proliferation, as indicated by reduced tritiated thymidine uptake.

In accordance with this invention, there is provided a new immunosuppressant, "Tsukubamycin A", which exhibits positive inhibition of T-cell activation by the T-cell proliferation assay and exhibits a high resolution mass spectral molecular ion of (m/z) 1093.6556, as the tetra (trimethylsilyl) derivative and characteristic fragment ions at m/z 673 and 481.

Also provided is a pharmaceutical composition containing a therapeutically effective amount of "Tsukubamycin A" in combination with a pharmaceutically acceptable, substantially non-toxic carrier or excipient.

In addition, there is provided a method of use for treating human host to prevent transplantation rejection, or for treating autoimmune disease or infectious disease comprising administering to said host a therapeutically

effective amount of "Tsukubamycin A".

Furthermore, there is provided a new process for producing the immunosuppressant, "Tsukubamycin B", which exhibits positive inhibition of T-cell activation by the T-cell proliferation assay and exhibits a high resolution mass spectral molecular ion of (m/z) 1021.6169, as the tri-(trimethylsilyl) derivative and characteristic fragment ions at m/z 675 and 483, comprising the step of fermenting the microorganism Streptomyces tsukubaensis No. 9993, (FERM BP-927) under submerged aerobic conditions in an aqueous carbohydrate medium, containing a nitrogen nutrient, said conditions being conducted at a pH of about 7, and recovering said "Tsukubamycin B".

## DETAILED DESCRIPTION OF THE INVENTION AND PREFERRED EMBODIMENTS

The present invention involves the fermentation of Streptomyces tsukubaensis No. 9993, (FERM BP-927) to produce "Tsukubamycins A and B". The microorganism, including physical characteristics and taxonomy, including morphological, cultural, biological and physiological characteristics are described in EPO Publication No. 0184162 to Fujisawa.

In general, "Tsukubamycins A and B" can be produced by culturing (fermenting) Streptomyces tsukubaensis No. 9993 (FERM BP-927) in an aqueous nutrient medium containing sources of assimilable carbon and nitrogen, preferably under submerged aerobic conditions (e.g. shaking culture, submerged culture, etc.). The aqueous medium is preferably maintained at a pH of about 7 at the initiation and termination (harvest) of the fermentation process. A higher pH leads to substantial and/or total loss of product.

The desired pH may be maintained by the use of a buffer such as morpholinoethanesulfonic acid (MES), morpholinopropanesulfonic acid (MOPS), and the like, or by choice of nutrient materials which inherently possess buffering properties, such as production media described hereinbelow.

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, meat extract, peptone, gluten meal, cottonseed meal, soybean meal and other vegetable meals (partially or totally defatted), casein hydrolysates, soybean hydrolysates and yeast hydrolysates, corn steep liquor, dried yeast, wheat germ, feather meal, peanut powder, distiller's solubles, etc., as well as inorganic and organic nitrogen compounds such as ammonium salts (e.g. ammonium nitrate, ammonium sulfate, ammonium phosphate, etc.), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts, and the like. If necessary, especially when the culture medium foams seriously, a defoaming agent, such as liquid paraffin, fatty oil, plant oil, mineral oil or silicone may be added.

As to the conditions for the production of "Tsukubamycins A and B" in massive amounts, submerged aerobic culturing conditions are preferred therefor. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production of "Tsukubamycins A and B". Accordingly, it is desirable first to produce a vegetative inoculum of the organism by inoculating a relatively small quantity of culture medium with spores or mycelia of the organism produced in a "slant" and culturing said inoculated medium, also called the "seed medium", and then to transfer the cultured vegetative inoculum aseptically to large tanks. The fermentation medium, in which the inoculum is produced, is substantially the same as or different from the medium utilized for the production of "Tsukubamycins A and B" and is generally autoclaved to sterilize the medium prior to inoculation. The pH of the medium is generally adjusted to about 7.0 prior to the autoclaving step by suitable addition of an acid or base, preferably in the form of a buffering solution.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

Preferred culturing/production media for carrying out the fermentation include the following media:

Fermentation Seed Medium FK-S1

| | |
|---|---|
| Cerelose | 5.5 g/l |
| Glycerol | 10.5 g/l |
| Corn Starch 3005 | 10.0 g/l |
| Pharmamedia | 10.0 g/l |
| Corn Steep Liquor | 5.0 g/l |
| Calcium Carbonate | 2.0 g/l |
| Polyglycol P-2000 | 1.0 ml/l |

Fermentation Production Medium FK-P1

| | |
|---|---|
| Soluble Starch | 45.0 g/l |
| Corn Steep Liquor | 10.0 g/l |
| Primary Dry Yeast | 10.0 g/l |
| Calcium Carbonate | 1.0 g/l |
| Polyglycol P-2000 | 2.0 ml/l |

The produced "Tsukubamycins A and B" can be recovered from the culture medium by conventional means which are commonly used for the recovery of other known biologically active substances. The "Tsukubamycins A and B" substances produced are found in the cultured mycelium and filtrate, and accordingly can be isolated and purified from the mycelium and the filtrate, which are obtained by filtering or centrifuging the cultured broth, by a conventional method such as concentration under reduced pressure, lyophilization, extraction with a conventional solvent, such as methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methanol.

The products "Tsukubamycins A and B" from the fermentation exhibit positive immunosuppressive activity in the "T-cell proliferation assay" and possesses utility on this basis.

The product "Tsukubamycin A" exhibits the following physical characteristics:

1. White amorphous powder
2. Solubility in methanol
3. Molecular weight of 805, as determined by mass spectrometry consistent with a molecular ion at (m/z) 1093.6556 for the tetra-(trimethylsilyl) derivative and characteristic fragment ions at (m/z) 673 and 481.

The product "Tsukubamycin B" exhibits the following physical characteristics:

1. White amorphous powder
2. Solubility in methanol
3. Molecular weight of 805, as determined by mass spectrometry consistent with a molecular ion at (m/z) 1021.6169 for the tri-(trimethylsilyl) derivative and characteristic fragment ions at (m/z) 675 and 483.

The "Tsukubamycins A and B" obtained according to the fermentation processes as explained above can be isolated and purified in a conventional manner, for example, extraction, precipitation, fractional crystallization, recrystallization, chromatography, and the like.

Suitable salts of the material may include pharmaceutically acceptable salts such as basic salts, for example, alkali metal salt (e.g. sodium salt, potassium salt, etc.), alkaline earth metal salt (e.g. calcium salt, magnesium salt, etc.), ammonium salt, amine salt (e.g. triethylamine salt, N-benzyl-N-methylamine salt, etc.) and other conventional organic salts.

The "Tsukubamycins A and B" of the present invention possesses pharmacological activity such as immunosuppressive activity, antimicrobial activity, and the like, and therefore is useful for the treatment and prevention of the transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosus, Hashimoto's thyroiditis, multiple sclerosis, myasthenia gravis, type I diabetes, uveitis, and the like.

The pharmaceutical composition of this invention can be used in the form of a pharmaceutical preparation, for example, in solid, semisolid or liquid form, which contains the "Tsukubamycins A and B", of the present invention, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, it preferable to apply it by parenteral or enteral administration. While the dosage of therapeutically effective amount of the "Tsukubamycins A and B" varies from, and also depends upon the age and condition of each individual patient to be treated, a daily dose (calculated on the basis of a 70 kg man) of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The following examples are given for the purpose of illustrating the present invention and should not be

construed as being limitations on the scope or spirit of the instant invention.

## EXAMPLE 1

### Fermentation

A seed culture was produced by inoculation of 40 ml of FK-S1 media in a triple baffled Erlenmeyer flask with a slant of culture MA-6492 (S. tsukubaensis No. 9993). The inoculated medium was incubated for 72 hours at 29.0° C on a 220 rpm shaker.

Several triple baffled 2 liter Erlenmeyer flasks containing 250 ml of FK-S1 medium were inoculated with 2.5 ml of the resulting culture. The inoculated medium was incubated for 24 hours at 29.0° C on a 220 rpm shaker.

Seven hundred fifty ml of the resulting culture was then inoculated into 180 liters of FK-S1 medium contained in a 300 liter, stainless steel, agitated fermentor. The inoculated medium was incubated for 24 hours at 29.5° C with 120 rpm agitation, 160 lpm air flow and 0.7 kg/cm$^2$ back pressure.

A 19,000 liter stainless steel fermentor containing 13,000 liters of FK-P1 medium agitated with four hydrofoil impellers was inoculated with 130 liters of the resulting culture. Fermentation was carried out for 104 hours at 29.5° C with 100 rpm agitation, 10.5 KL/min air flow and 0.7 kg/cm$^2$ back pressure. The dissolved oxygen concentration was maintained above 50% of air saturation.

## EXAMPLE 2

### Isolation and Purification Procedure from Broth

Methanol (6000 liters) was added to whole broth (12,000 liters) with vigorous agitation. The resulting suspension was freed of solids by means of a disk centrifuge. The centrifuged aqueous methanolic extract was then passed through two columns (45 x 180 cm, approximately 285 liters each) of DIAION HP-20 (Mitsubishi Chemicals) run in parallel. Each column was eluted with methanol (1600 liters), and fractions containing the object compound were pooled to afford a composite richcut (1400 liters). The richcut was concentrated in vacuo to a final volume of 80 liters.

Water (320 liters) was added to the concentrate, which was then extracted with two portions of ethyl acetate (each 200 liters). The pooled ethyl acetate extracts were then concentrated in vacuo to afford a heavy oil (8 liters).

The concentrated ethyl acetate extract was diluted with hexane (4 liters) and chromatographed on a column (30 x 90 cm, approximately 35 liters) of silica gel (60-200 mesh, W.R. Grace Co.) in hexane. The column was first washed with hexane (200 liters) then eluted with hexane:acetone (3:1, 400 liters). Fractions containing the object compound were pooled and concentrated in vacuo to afford a viscous oil.

Further purification was achieved via preparative reverse-phase high performance liquid chromatography (HPLC). The richcut was chromatographed on a Dynamax-60A C18 column (21.4 x 250 mm, Rainin Instruments), operated at 55° C. The column was eluted with acetonitrile:water (3:2) at 9.9 ml/minute. Elution was monitored for UV absorbance at 205 nanometers. The compound was collected during repeated injection of the above described extract. Fractions containing the object compound were pooled and concentrated in vacuo, then repeatedly rechromatographed under the same conditions.

Fractions containing the object compound were analyzed by reverse-phase HPLC using a Partisil-5 ODS3 column (4.6 x 250 mm, Whatman) operated at 56° C and monitored for ultraviolet absorbance at 205 nanometers. The column was eluted isocratically with acetonitrile:water (13:7) at 1.5 ml/minute. Under these conditions, the object compound, designated "Tsukubamycin A" (1.27 grams), has a retention time of 7.36 minutes, differing from Fujisawa's FR-900506, FR-900520 and FR-900523, as described in EPO Publication No. 0184162.

"Tsukubamycin A" was characterized by high resolution mass spectrometry of its tetra-(trimethylsilyl)-derivative. A molecular ion at m/z 1093.6556 (tetra-(trimethylsilyl) derivative of $C_{44}H_{71}NO_{12}$ calculated 1093.6557) and characteristic fragment ions at m/z 673 and m/z 481 were observed.

## EXAMPLE 3

### Isolation and Purification Procedure from Broth

Whole broth (12,000 liters) was centrifuged with a disk centrifuge affording a concentrated suspension of the culture solids (2000 liters). Methanol (2000 liters) was added to this suspension, with vigorous agitation. The extract was freed of solids via a disk centrifuge, and the resulting solids were re-extracted with methanol:water (1:1, 2000 liters). After removal of the solids using a disk centrifuge, the two aqueous methanolic extracts were pooled and freed of solvent in vacuo to afford 2000 liters of an aqueous

concentrate. The aqueous concentrate was extracted with two portions of ethyl acetate (each 1000 liters). The pooled ethyl acetate extracts were freed of solvent in vacuo to afford a heavy oil (8 liters).

The concentrated ethyl acetate extract was diluted with hexane (4 liters) and chromatographed on a column (30 x 90 cm, approximately 35 liters) of silica gel (60-200 mesh, W.R. Grace Co.) in hexane. The column was first washed with hexane (200 liters) then eluted with hexane:acetone (3:1, 400 liters). Fractions containing the object compound were pooled and concentrated to afford a viscous oil.

Further purification was achieved via preparative reverse-phase high performance liquid chromatography (HPLC). The richcut was chromatographed on a Dynamax-60A C18 column (21.4 x 250 mm, Rainin Instruments), operated at 55°C. The column was eluted with acetonitrile:water (3:2) at 9.9 ml/minute. Elution was monitored for UV absorbance at 205 namometers. The compound was collected during repeated injection of the above described extract. Fractions containing the object compound were pooled and concentrated in vacuo, then repeatedly rechromatographed under the same conditions.

Fractions containing the object compound were analyzed by reverse-phase HPLC using a Partisil-5 ODS3 column (4.6 x 250 mm, Whatman) operated at 56°C and monitored for ultraviolet absorbance at 205 nanometers. The column was eluted isocratically with acetonitrile:water (13:7) at 1.5 ml/minute. Under these conditions, the object compound, designated "Tsukubamycin A" (1.27 grams), had a retention time of 7.36 minutes, differing from Fujisawa's FR-900506, FR-900520 and FR-900523, as described in EPO Publication No. 0184162.

"Tsukubamycin A" was characterized by proton and $^{13}C$ nuclear magnetic spectroscopy and by high resolution mass spectrometry of its tetra-(trimethylsilyl)-derivative. A molecular ion at m/z 1093.6556 (tetra(trimethylsilyl) derivative of $C_{44}H_{71}NO_{12}$, calculated 1093.6557) and characteristic fragment ions at m/z 673 and m/z 481 were observed.

## EXAMPLE 4

### T-Cell Proliferation Assay

1. Sample Preparation

"Tsukubamycin A", as prepared by HPLC above, was dissolved in absolute ethanol.

2. Assay

Spleens from C57B1/6 mice were taken under sterile conditions and gently dissociated in ice-cold RPMI 1640 culture medium (GIBCO, Grand Island, N.Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent T lymphocytes were eluted from the columns with warm culture medium and the cell suspensions were spun as above.

Purified T lymphocytes were resuspended at $2.5 \times 10^5$ cells/ml in complete culture medium composed of RPMI 1640 medium with 10% heat-inactivated fetal calf serum, 100 mM glutamine, 1 mM sodium pyruvate, $2 \times 10^{-5}$ M 2-mercaptoethanol and 50 µg/ml gentamycin. Ionomycin was added at 250 ng/ml and PMA at 10 ng/ml. The cell suspension was immediately distributed into 96 well flat-bottom microculture plates (Costar) at 200 µl/well. The control, being the medium without test drug, and various below-indicated dilutions of the sample (above-described purified "Tsukubamycin A") to be tested were then added in triplicate wells at 20 µl/well. L-679,934 was used as a standard. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 µCi/well of tritiated thymidine (NEN, Cambridge, MA). After another 4 hours of incubation, cultures were harvested on glass fiber filters using a multiple sample harvester. Radioactivity of filter discs corresponding to individual wells was measured by standard liquid scintillation counting methods (Beta counter). Mean counts per minute of replicate wells were calculated and the results expressed as percent inhibition of tritiated thymidine uptake (proliferation) as follows:

$$\% \text{ Inhibition} = 100 - \left[\frac{\text{Mean cpm sample tested}}{\text{Mean cpm control medium}}\right] \times 100.$$

The results of % inhibition at various concentrations of "Tsukubamycin A" indicated its immunosuppressant properties.

Also, inhibition of T-cell proliferation by "Tsukubamycin A" was reversed by the addition of 50 units/ml of IL-2 (recombinant human IL-2) at the initiation of culture.

## EXAMPLE 5

Fermentation

A seed culture was produced by inoculation of 40 ml of FK-S1 media in a triple baffled Erlenmeyer flask with a slant of culture MA-6492 (S. tsukubaensis No. 9993). The inoculated medium was incubated for 72 hours at 29.0° C on a 220 rpm shaker.

Several triple baffled 2 liter Erlenmeyer flasks containing 250 ml of FK-S1 medium were inoculated with 2.5 ml of the resulting culture. The inoculated medium was incubated for 24 hours at 29.0° C on a 220 rpm shaker.

Seven hundred fifty ml of the resulting culture was then inoculated into 180 liters of FK-S1 medium contained in a 300 liter, stainless steel, agitated fermentor. The inoculated medium was incubated for 24 hours at 29.5° C with 120 rpm agitation, 160 lpm air flow and 0.7 $kg/cm^2$ back pressure.

A 19,000 liter stainless steel fermentor containing 13,000 liters of FK-P1 medium agitated with four hydrofoil impellers was inoculated with 130 liters of the resulting culture. Fermentation was carried out for 104 hours at 29.5° C with 100 rpm agitation, 10.5 KL/min air flow and 0.7 $kg/cm^2$ back pressure. The dissolved oxygen concentration was maintained above 50% of air saturation.

## EXAMPLE 6

Isolation and Purification Procedure from Broth

Methanol (6000 liters) was added to whole broth (12,000 liters) with vigorous agitation. The resulting suspension was freed of solids by means of a disk centrifuge. The centrifuged aqueous methanolic extract was then passed through two columns (45 x 180 cm, approximately 285 liters each) of DIAION HP-20 (Mitsubishi Chemicals) run in parallel. Each column was eluted with methanol (1600 liters), and fractions containing the object compound were pooled to afford a composite richcut (1400 liters). The richcut was concentrated in vacuo to a final volume of 80 liters.

Water (320 liters) was added to the concentrate, which was then extracted with two portions of ethyl acetate (each 200 liters). The pooled ethyl acetate extracts were then concentrated in vacuo to afford a heavy oil (8 liters).

The concentrated ethyl acetate extract was diluted with hexane (4 liters) and chromatographed on a column (30 x 90 cm, approximately 35 liters) of silica gel (60-200 mesh, W.R. Grace Co.) in hexane. The column was first washed with hexane (200 liters) then eluted with hexane:acetone (3:1, 400 liters). Fractions containing the object compound were pooled and concentrated in vacuo to afford a viscous oil.

Further purification was achieved via preparative reverse-phase high performance liquid chromatography (HPLC). The richcut was chromatographed on a Dynamax-60A C18 column (21.4 x 250 mm, Rainin Instruments), operated at 55° C. The column was eluted with acetonitrile:water (3:2) at 9.9 ml/minute. Elution was monitored for UV absorbance at 205 nanometers. The compound was collected during repeated injection of the above described extract. Fractions containing the object compound were pooled and concentrated in vacuo, then repeatedly rechromatographed under the same conditions.

Fractions containing the object compound were analyzed by reverse-phase HPLC using a Partisil-5 ODS3 column (4.6 x 250 mm, Whatman) operated at 56° C and monitored for ultraviolet absorbance at 205 nanometers. The column was eluted isocratically with acetonitrile:water (13:7) at 1.5 ml/minute. Under these conditions, the object compound, designated "Tsukubamycin B", has a retention time of 10.24 minutes, differing from Fujisawa's FR-900506, FR-900520 and FR-900523, as described in EPO Publication No. 0184162.

"Tsukubamycin B" was characterized by proton and $^{13}C$ nuclear magnetic resonance spectroscopy, and by high resolution mass spectrometry of its tri-(trimethylsilyl) derivative. A molecular ion at m/z 1021.6169 (tri-(trimethylsilyl) derivative of $C_{44}H_{71}NO_{12}$ calculated 1021.6162) and characteristic fragment ions at m/z 675 and m/z 483 were observed.

## EXAMPLE 7

Isolation and Purification Procedure from Broth

Whole broth (12,000 liters) was centrifuged with a disk centrifuge affording a concentrated suspension of the culture solids (2000 liters). Methanol (2000 liters) was added to this suspension, with vigorous agitation. The extract was freed of solids via a disk centrifuge, and the resulting solids were re-extracted with methanol:water (1:1, 2000 liters). After removal of the solids using a disk centrifuge, the two aqueous methanolic extracts were pooled and freed of solvent in vacuo to afford 2000 liters of an aqueous concentrate. The aqueous concentrate was extracted with two portions of ethyl acetate (each 1000 liters). The pooled ethyl acetate extracts were freed of solvent in vacuo to afford a heavy oil (8 liters).

The concentrated ethyl acetate extract was diluted with hexane (4 liters) and chromatographed on a column (30 x 90 cm, approximately 35 liters) of silica gel (60-200 mesh, W.R. Grace Co.) in hexane. The column was first washed with hexane (200 liters) then eluted with hexane:acetone (3:1, 400 liters). Fractions containing the object compound were pooled and concentrated to afford a viscous oil.

Further purification was achieved via preparative reverse-phase high performance liquid chromatography (HPLC). The richcut was chromatographed on a Dynamax-60A C18 column (21.4 x 250 mm, Rainin Instruments), operated at 55°C. The column was eluted with acetonitrile:water (3:2) at 9.9 ml/minute. Elution was monitored for UV absorbance at 205 namometers. The compound was collected during repeated injection of the above described extract. Fractions containing the object compound were pooled and concentrated in vacuo, then repeatedly rechromatographed under the same conditions.

Fractions containing the object compound were analyzed by reverse-phase HPLC using a Partisil-5 ODS3 column (4.6 x 250 mm, Whatman) operated at 56°C and monitored for ultraviolet absorbance at 205 nanometers. The column was eluted isocratically with acetonitrile:water (13:7) at 1.5 ml/minute. Under these conditions, the object compound, designated "Tsukubamycin B", had a retention time of 10.24 minutes, differing from Fujisawa's FR-900506, FR-900520 and FR-900523, as described in EPO Publication No. 0184162.

"Tsukubamycin B" was characterized by high resolution mass spectrometry of its tri-(trimethylsilyl) derivative. A molecular ion at m/z 1021.6169 (tri-(trimethylsilyl) derivative of $C_{44}H_{71}NO_{12}$ calculated 1021.6162) and characteristic fragment ions at m/z 675 and m/z 483 were observed.

## EXAMPLE 8

T-Cell Proliferation Assay

1. Sample Preparation

"Tsukubamycin B", as prepared by HPLC above, was dissolved in absolute ethanol.

2. Assay

Spleens from C57B1/6 mice were taken under sterile conditions and gently dissociated in ice-cold RPMI 1640 culture medium (GIBCO, Grand Island, N.Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent T lymphocytes were eluted from the columns with warm culture medium and the cell suspensions were spun as above.

Purified T lymphocytes were resuspended at 2.5 x $10^5$ cells/ml in complete culture medium composed of RPMI 1640 medium with 10% heat-inactivated fetal calf serum, 100 mM glutamine, 1 mM sodium pyruvate, 2 x $10^{-5}$ mercaptoethanol and 50 µg/ml gentamycin. Ionomycin was added at 250 ng/ml and PMA at 10 ng/ml. The cell suspension was immediately distributed into 96 well flat-bottom microculture plates (Costar) at 200 µl/well. The control, being the medium without test drug, and various below-indicated dilutions of the sample (above-described "Tsukubamycin B") to be tested were then added in triplicate wells at 20 µl/well. FK-506 was used as a standard. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 µCi/well of tritiated thymidine (NEN, Cambridge, MA). After another 4 hours of incubation, cultures were harvested on glass fiber filters using a multiple sample harvester. Radioactivity of filter discs corresponding to individual wells was measured by standard liquid scintillation counting methods (Beta counter). Mean counts per minute of replicate wells were calculated and the results expressed as percent inhibition of tritiated thymidine uptake (proliferation) as follows:

$$\% \text{ Inhibition} = 100 - \left[\frac{\text{Mean cpm sample tested}}{\text{Mean cpm control medium}}\right] \text{X } 100.$$

The results of % inhibition at various concentrations of "Tsukubamycin B" indicated its immunosuppressant properties.

Also, inhibition of T-cell proliferation by "Tsukubamycin B" was reversed by the addition of 50 units/ml of IL-2 (recombinant human IL-2) at the initiation of culture.

## Claims

1. An immunosuppressant, "Tsukubamycin A", which exhibits positive inhibition of T-cell activation by the T-cell proliferation assay, and exhibits a high resolution mass spectral molecular ion of (m/z) 1093.6556, as the tetra-(trimethylsilyl) derivative, with characteristic fragment ions at m/z 673 and 481.

2. A pharmaceutical composition containing a therapeutically effective amount of "Tsukubamycin A" in combination with a pharmaceutically acceptable, substantially non-toxic carrier or excipient.

3. The use of "Tsukubamycin A", for the preparation of a medicament useful for preventing transplantation rejection, or for treating autoimmune disease or infectious disease.

4. A process for producing the immunosuppressant, "Tsukubamycin B", being the C-21 propyl analog of FK-506, which exhibits positive inhibition of T-cell activation by the T-cell proliferation assay, and exhibits a high resolution mass spectral molecular ion of (m/z) 1021.6169, as the tri-(trimethylsilyl) derivative, with characteristic fragment ions at m/z 675 and 483, comprising the step of fermenting the microorganism Streptomyces tsukubaensis No. 9993. (FERM BP-927) under submerged aerobic conditions in an aqueous carbohydrate medium, containing a nitrogen nutrient, said conditions being conducted at a pH of about 7, and recovering said "Tsukubamycin B".

"TSUKUBAMYCIN A"

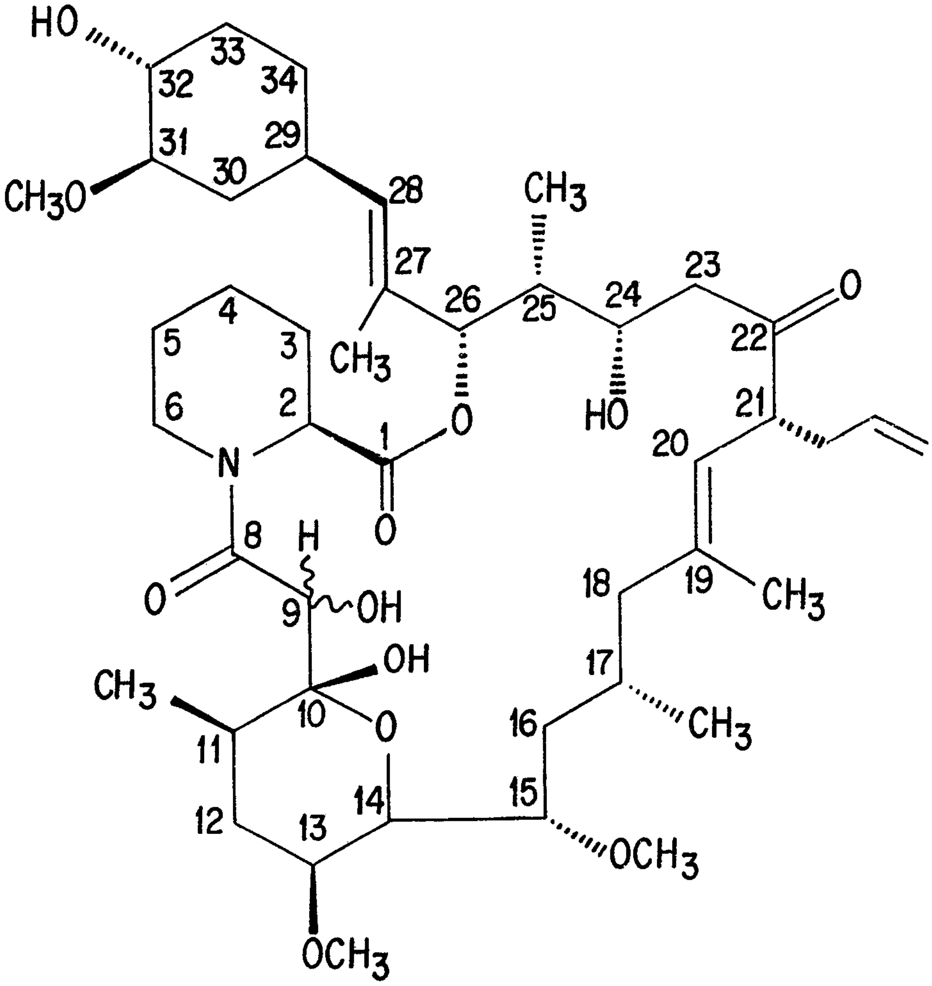

FIG. I

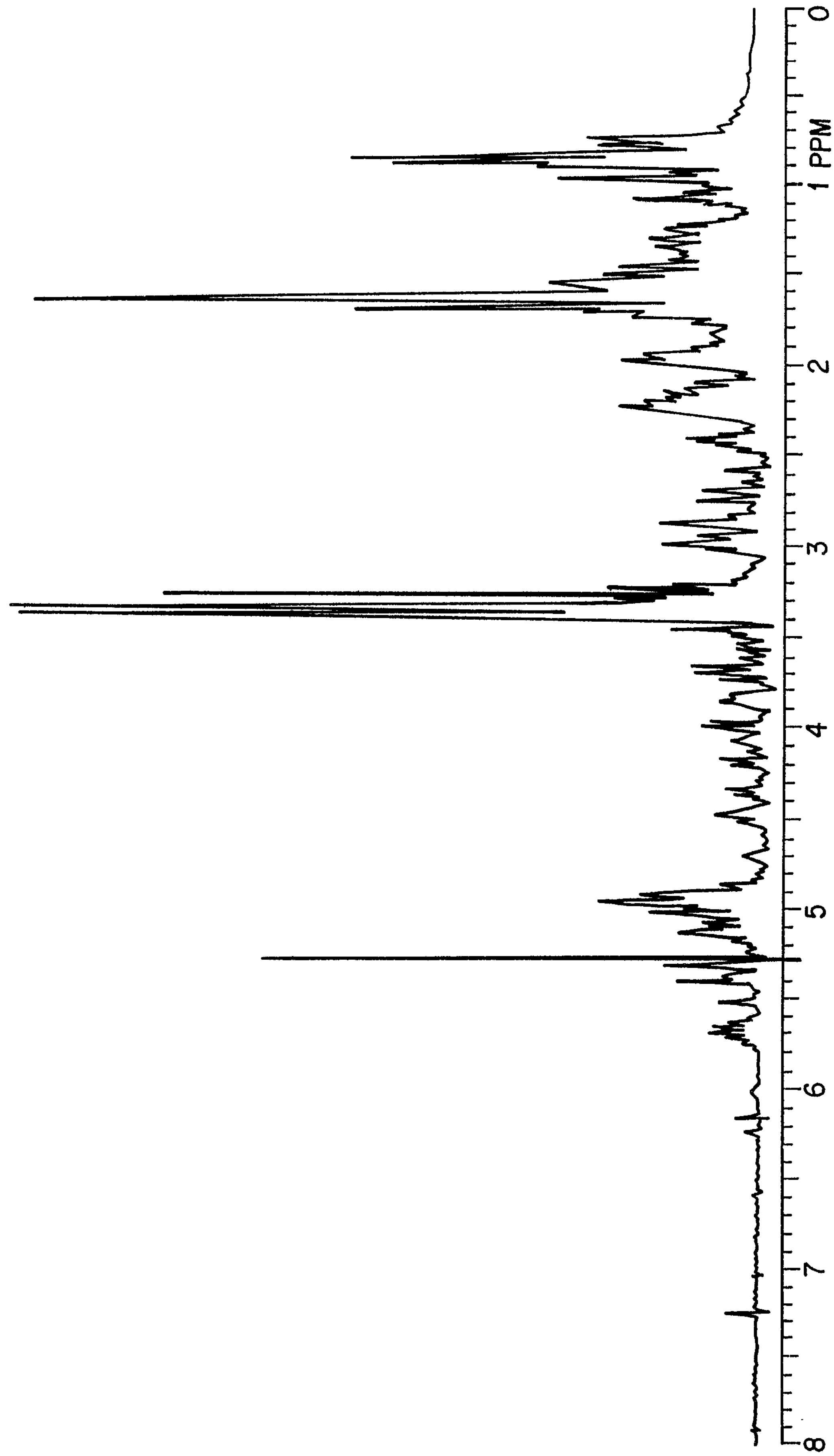


FIG. 2

"TSUKUBAMYCIN B"

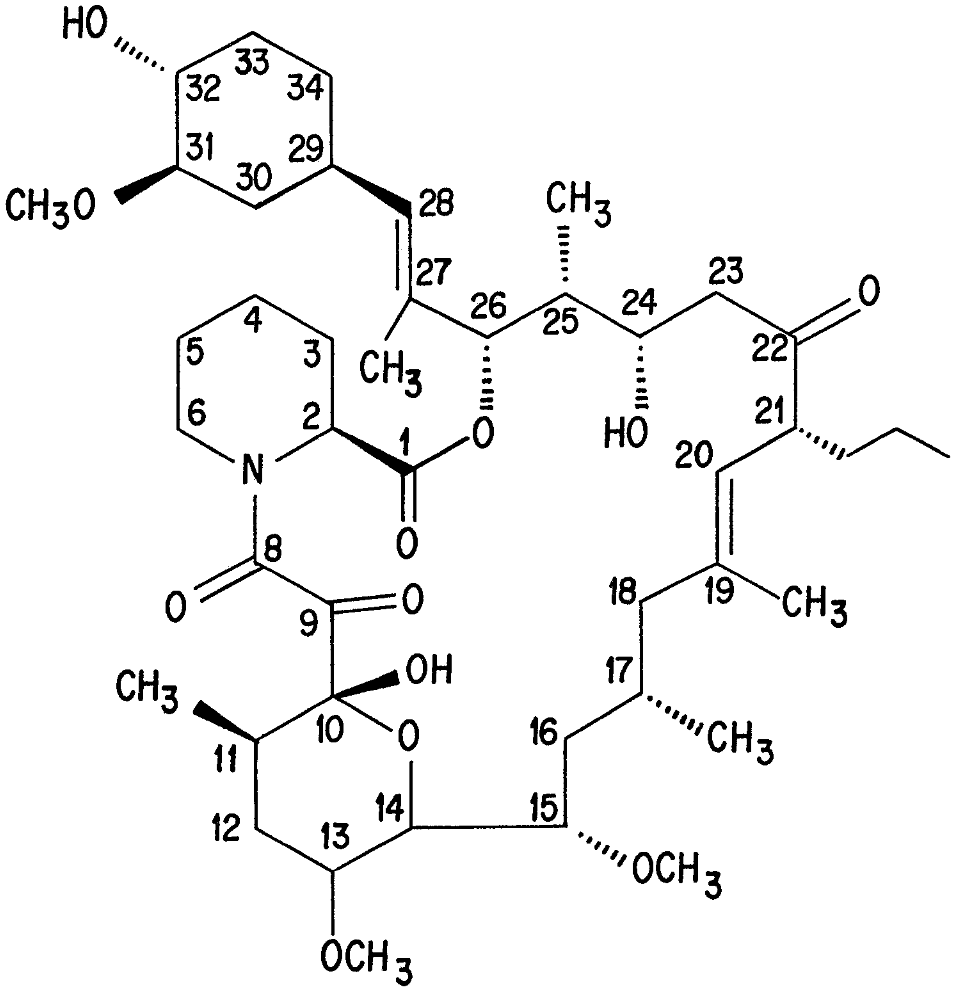

FIG. 3

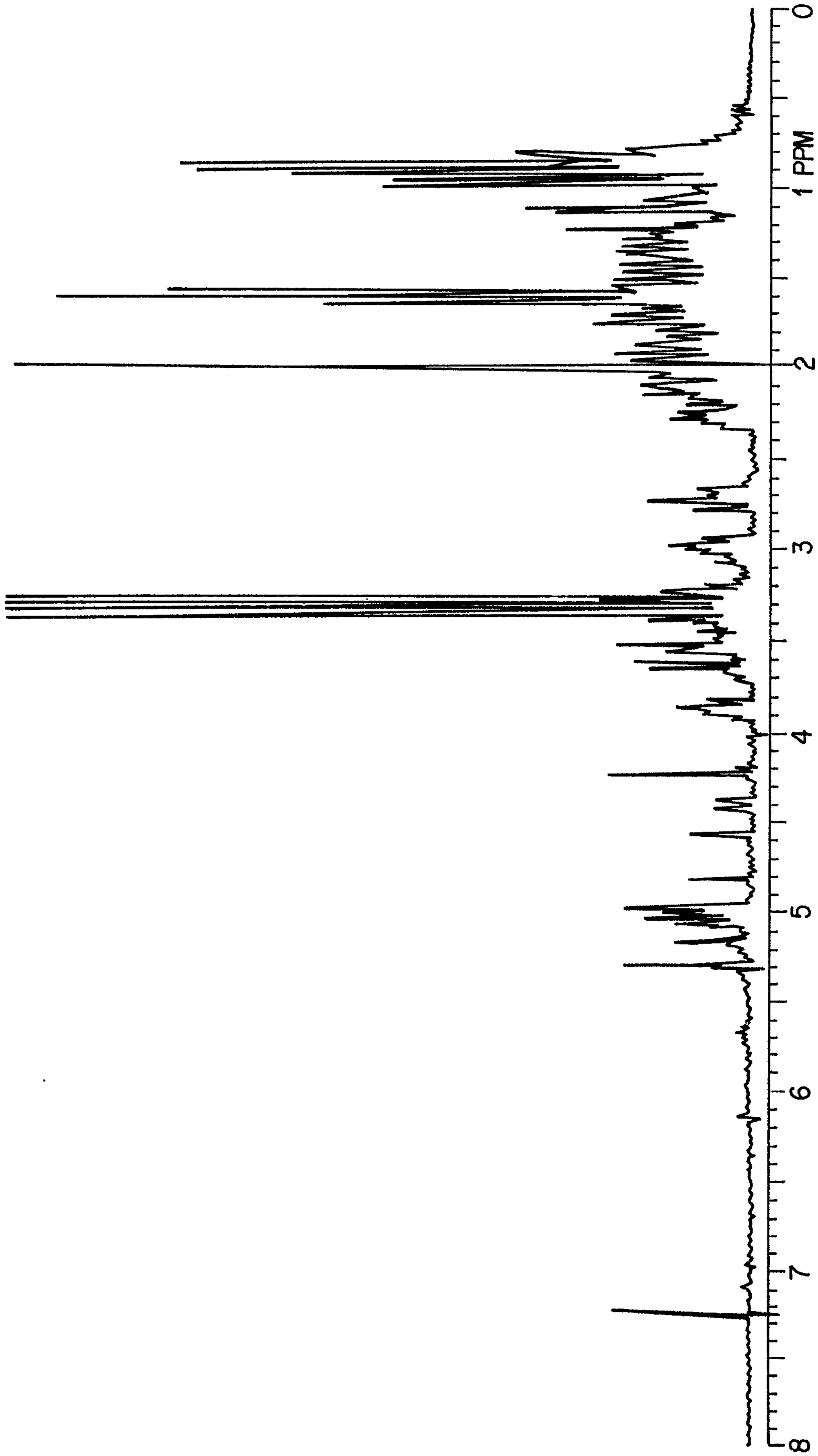


FIG. 4

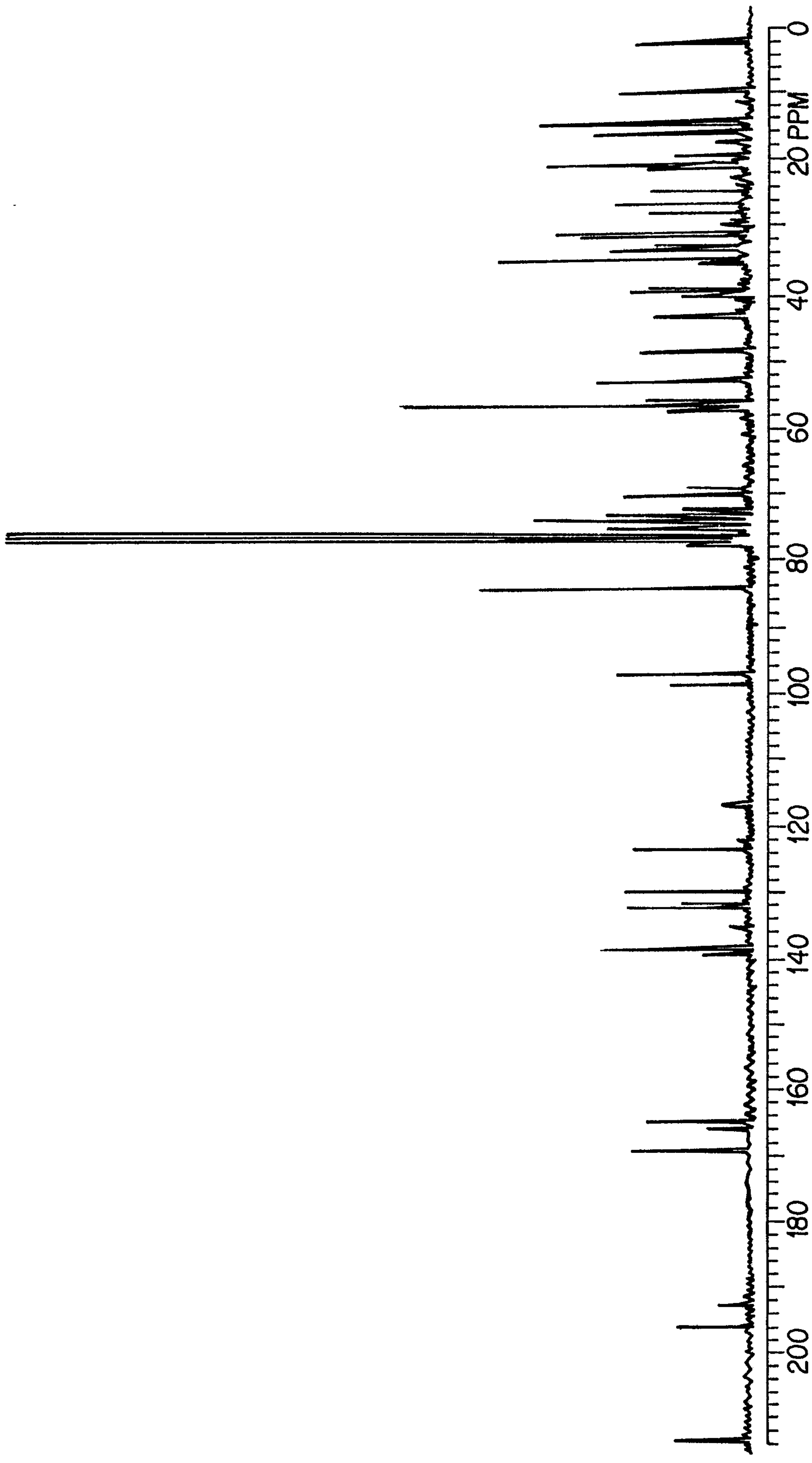


FIG. 5